(19) Europäisches Patentamt
European Patent Office
Office européen des brevets

(11) Publication number: **0 044 292**
**B1**

(12) **EUROPEAN PATENT SPECIFICATION**

(45) Date of publication of patent specification: **17.10.84**

(51) Int. Cl.$^3$: **G 02 B 27/42,** A 61 B 6/02

(21) Application number: **80900162.1**

(22) Date of filing: **10.01.80**

(86) International application number: **PCT/NL80/00001**

(87) International publication number: **WO 81/01952 23.07.81 Gazette 81/17**

(54) **METHOD OF AND DEVICE FOR CODING AND DECODING AN IMAGE OF AN OBJECT BY MEANS OF PENETRATING RADIATION.**

(43) Date of publication of application: **27.01.82 Bulletin 82/04**

(45) Publication of the grant of the patent: **17.10.84 Bulletin 84/42**

(84) Designated Contracting States: **DE FR GB NL SE**

(56) References cited:
**FR-A-2 320 574**
**GB-A-2 019 599**

**IRE Transactions on Information Theory, volume 7, published April 1961, (New York, US), M. Golay "Complementary series", pages 82-87**
**IEEE Transactions on Nuclear Science, volume NS-25, no. 1, published February 1978, (New York, US), T. Cannon et al. "A class of near perfect coded apertures", pages 184-188**

(73) Proprietor: **N.V. Philips' Gloeilampenfabrieken**
**Groenewoudseweg 1**
**NL-5621 BA Eindhoven (NL)**
(73) Proprietor: **Philips Patentverwaltung GmbH**
**Billstrasse 80**
**D-2000 Hamburg 28 (DE)**

(72) Inventor: **WEISS, Herman**
**Marderstrasse 60**
**D-2000 Hamburg 65 (DE)**
Inventor: **PASEDACH, Klaus**
**Karstenstrasse 5**
**D-2000 Hamburg 55 (DE)**

(74) Representative: **Faessen, Louis Marie et al**
**INTERNATIONAAL OCTROOIBUREAU B.V. 6**
**Prof. Holstlaan**
**NL-5656 AA Eindhoven (NL)**

**EP 0 044 292 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a method of coding and decoding an image of an object by means of radiation which is emitted by point sources and which irradiates the object in different directions in order to form shadow images of the object which are superposed on a recording medium, the coded image thus formed being decoded by convolution of the superposed shadow images with a function which is determined by the point source distribution used for irradiating the object.

The invention furthermore relates to a device for coding and decoding an image of an object by means of penetrating radiation, said device comprising for the coding of an image of an object a two-dimensional distribution of point sources and a two-dimensional image recording medium which are situated in two parallel planes at a distance from each other in order to form superposition images of the object, said device comprising for the decoding of the superposition images means for convoluting the recorded superposition images with at least a part of an image point function which is defined by the point source distribution, and means for summing the convoluted superposition images.

It is known that objects can be coded by irradiating the object from different positions by means of X-rays or other incoherent radiation and by recording the shadow images on a recording medium. Thus, a superposition image is obtained in which the information concerning the object is not directly accessible. The object or a layer object can be made visible again only during a second phase, *i.e.* the decoding of the superposition image. A method and a device of the described kind are known from German Patent Application No. P 25.35.408.9 (PHD 75—122c).

The decoding by means of the known specific arrays of the recording sources using incoherent or also coherent point distributions produces artefacts which prevent the signal-to-noise ratio from exceeding a given threshold.

The invention has for its object to provide a method of and a device for the coding and decoding of images of the described kind in which the object is decoded without disturbing artefacts.

To this end, a method in accordance with the invention is characterized in that for the coding the point sources are arranged according to a regular matrix, at least three different groups of point sources being used to form a corresponding number of superposition images in succession, a combination of groups forming a first source distribution function and a further combination of groups forming a second source distribution function, the sum of the autocorrelation functions of said source distribution functions having the value zero except in one point (origin), and for the decoding the different superposition images, or combinations with the negatives thereof, are convoluted with image point functions of the groups of sources in order to obtain convoluted images which are summed in order to obtain a decoded image. The combinations of groups which produce a first and a second source distribution function having the said property (the sum of the autocorrelation functions is zero with the exception of a value in one point (the origin)) are referred to as complementary combinations or complementary source distribution functions.

In the case of self-radiating objects, the array of radiation sources is effectively replaced by a corresponding array of diaphragms with holes wherethrough the radiation emitted by the object passes and is incident on a recording medium. The method can be used for two-dimensional as well as three-dimensional objects. In the case of three-dimensional objects, the reconstruction of the individual layers can be continuously performed by changing the scale of all superposition images on the recording means.

The artefact-free decoding of a layer may be understood to mean that the radiation sources are sub-divided into groups of each time positive sources (recording on a positive film) and negative sources (recording on a negative film), so that the sum of the autocorrelation of the distributions in the origin has a high value, corresponding to the sum $n$ of all sources, and that no secondary points occur in the sum of the autocorrelations of the two point source distributions. The decoded image is produced exactly with a mean intensity which is proportional to the value of the autocorrelation sum in the origin. It is to be noted that one-dimensional complementary codes have the arithmetical property that the sum of their autocorrelation consists of a maximum without secondary points (see IRE Transactions on Information Theory, Vol. 7, pages 82—87, 1961, M. J. E. Golay). The invention, however, is based on the use of complementary two-dimensional point codes, the two coherent codes being different and complementary in a sense that the sum of their autocorrelation has the previously described properties.

A device in accordance with the invention is characterized in that for the coding the device comprises means for the sequential activation of the point sources in at least three and at the most four different groups in order to obtain at least three coded images, a first number of groups of point sources forming a first distribution function and a second number of groups forming a second distribution function, the sum of the autocorrelations of said distribution functions having the value zero except in one point (origin), for the decoding the device comprising at least three processing channels for convoluting, either sequentially or simultaneously, the coded images with corresponding image point functions associated with the coded images, each of which is determined by a different group of point sources.

The decoding of the object can be performed by means of a computer, by analog electronic or by optical methods. The optical decoding can also be realized by means of a four-channel optical system, as will be described hereinafter.

The invention will be described in detail hereinafter with reference to the accompanying diagrammatic drawing.

Figure 1a) shows complementary point codes of the order 4, Fig. 1b) the autocorrelation of the two codes and their sum,

Figures 2*a* and *b* show a complementary code of the order 16 and different distributions of the points in 4 groups, respectively,

Figure 3 shows complementary codes of the order *n, n* having the value 1, 2, 4, 8 and 16,

Figure 4 shows the combination of codes of lower order in order to form codes of higher order,

Figure 5 shows complementary codes of the order *n, n* having the value 3/2, 5 and 10,

Figures 6*a*—*d* show radiation source distributions according to the complementary codes of the order *n, n* having the value 3/2, 5, 10 and 20, with code rotation and overlapping combination,

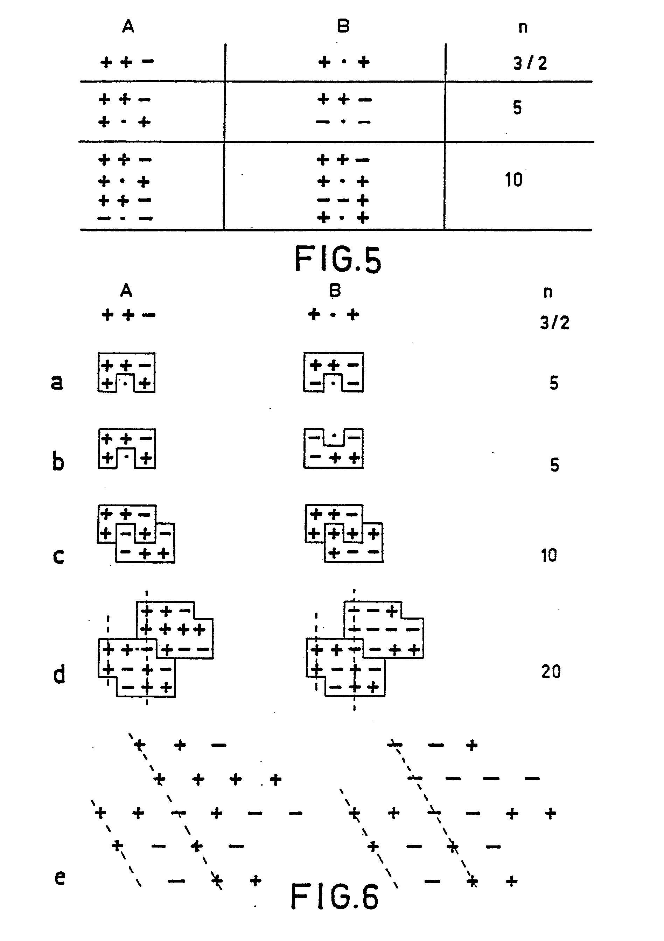

Figure 6*e* shows a slanted radiation source distribution,

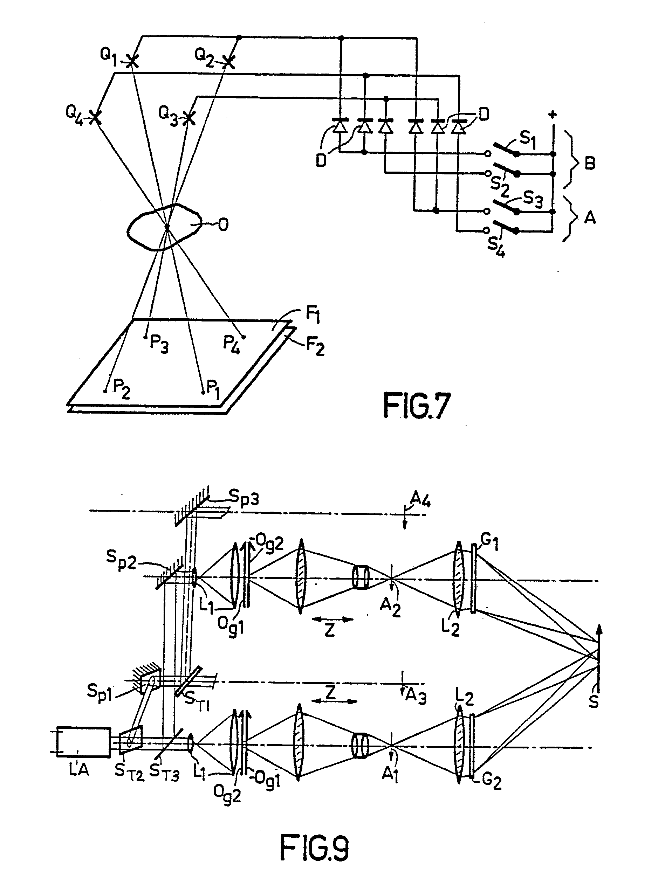

Figure 7 shows an arrangement for forming coded images,

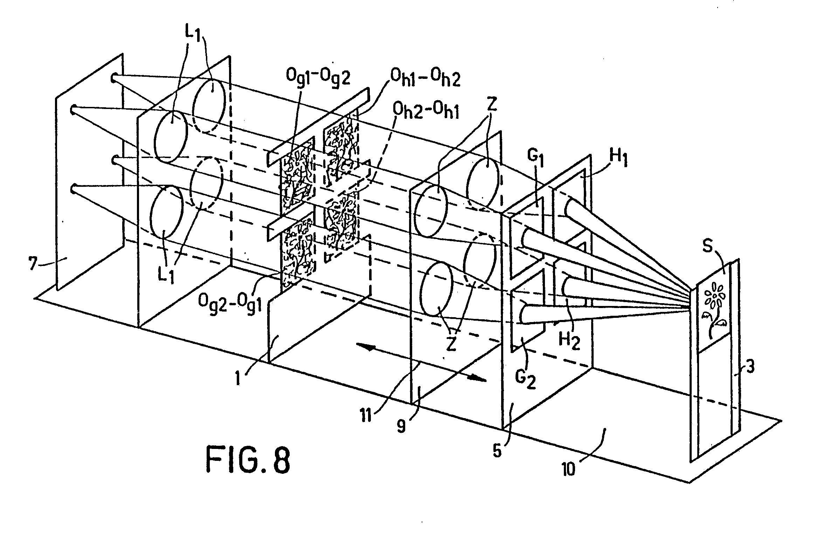

Figure 8 shows an arrangement for decoding the coded images obtained by means of the arrangement shown in Figure 7,

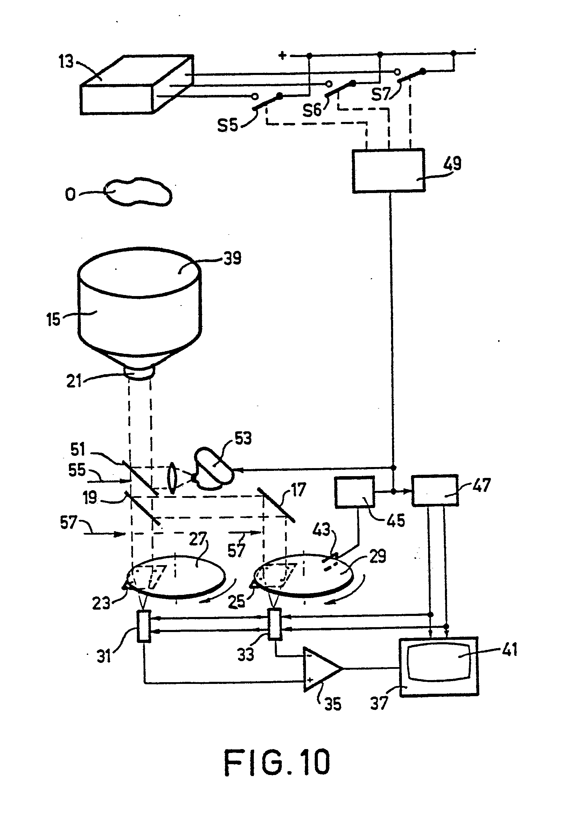

Figure 9 shows a part of the optical system of the arrangement shown in Figure 8,

Figure 10 shows an electro-optical device for the coding and the immediate decoding of a layer of the object, and

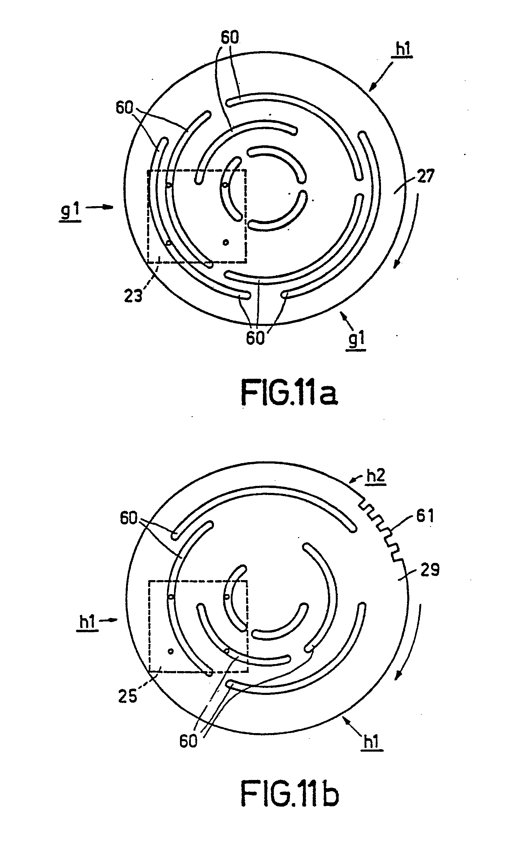

Figure 11 is a detailed view of a part of the arrangement shown in Figure 10.

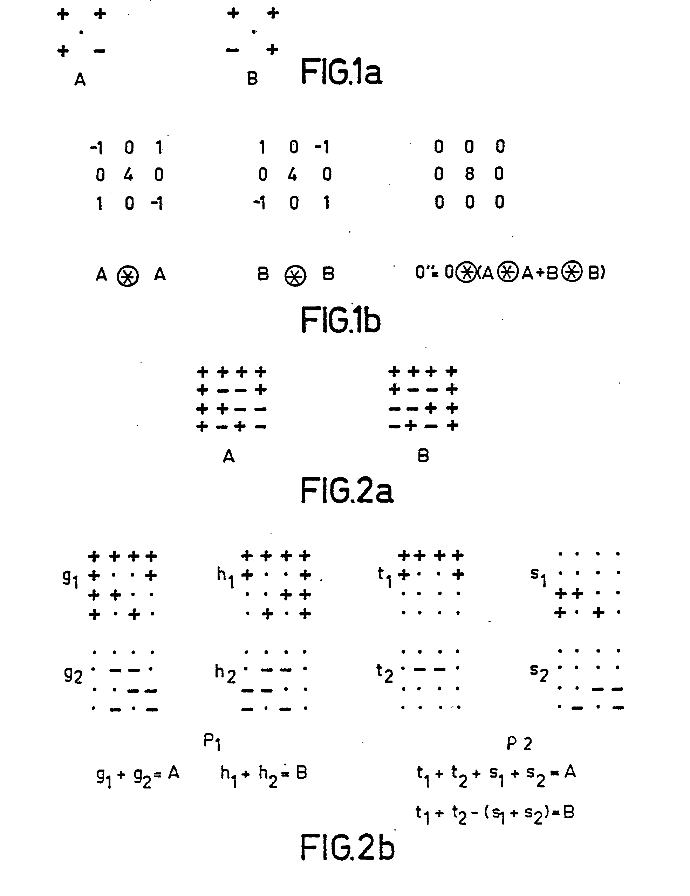

Figure 1 illustrates the principle on the basis of a pair of complementary point distributions (codes A, B with points + and —). An object O is recorded once by means of a point distribution with the code A, so that a coded image O′ of the object O is formed according to the correlation

$$O'_A = O * A \qquad (1)$$

$*$ means "convolution".

The object O is recorded a second time with the point source distribution B, so that the coded image:

$$O'_B = O * B \qquad (2)$$

is formed.

Each of these two coded images is then subjected to a correlation with the point image distribution function A and B, respectively, resulting in

$$O''_A = O * A \circledast A \qquad (3)$$

$$O''_B = O * B \circledast B \qquad (4)$$

When the images $O''_A$ and $O''_B$ thus obtained are added, the following is obtained

$$O'' = O''_A + O''_B = O * (A \circledast A + B \circledast B) \qquad (5)$$

Figure 1b shows the autocorrelations of the point source distributions A and B and the sum of both autocorrelations. The distributions A and B are chosen so that the sum of their autocorrelation has a value only in the origin M, so arithmetically it is a $\delta$-function, so that the equation (5) can be rewritten as:

$$O'' = O * \delta = O \qquad (6)$$

Formula (6) demonstrates that the object has been faithfully reconstructed.

Figure 2 shows an example of the coding of the object, a recording being made, for example, on X-ray film by means of X-rays.

The complementary point source distributions are of the order 16 in Figure 2a and can be subdivided into four groups in a different manner before the recording. The distributions chosen by way of example are shown in Figure 2b.

The distribution $P_1$ is based on the fact that each time the code point distributions A and B are separately subdivided, that is to say four recordings of the object O are made, each time with the point source distribution $g_1$, $g_2$, $h_1$ and $h_2$; $g_2$ and $h_2$ being "negative" sources. This means that the shadow images formed by means of $g_2$ and $h_2$ are subtracted from the shadow images formed by means of $g_1$ and $h_1$, respectively. Coded images according to (1) and (2) are then obtained

$$O'_A = O * g_1 - O * g_2 = O_{g1} - O_{g2} \qquad (7)$$

$$O'_B = O * h_1 - O * h_2 = O_{h_1} - O_{h_2}) \qquad (8)$$

in which $g_1$, $g_2$, $h_1$, $h_2$ each mark the distribution of the X-ray tubes which are simultaneously flashed.

Decoding is realized by the correlation according to (3) and (4)

$$\begin{aligned} O'' &= (O_{g_1} - O_{g_2}) \circledast (g_1 - g_2) + (O_{h_1} - O_{h_2}) \circledast (h_1 - h_2) \\ &= (O_{g_1} - O_{g_2}) \circledast g_1 + (O_{g_2} - O_{g_1}) \circledast g_2 + \\ &\quad (O_{h_1} - O_{h_2}) \circledast h_1 + (O_{h_2} - O_{h_1}) \circledast h_2 \end{aligned} \qquad (9)$$

The four additions of the equation (9) can be realized, for example, in four optical channels, as will be described hereinafter. However, from the coded image $O_{g_1}$, $O_{g_2}$, $O_{h_1}$, $O_{h_2}$ negatives

also have to be made and hence the combinations $O_{g_1}-O_{g_2}$, $O_{g_2}-O_{g_1}$, $O_{h_1}-O_{h_2}$, $O_{h_2}-O_{h_1}$. The combinations are convoluted with the point distributions $g_1$, $g_2$, $h_1$, $h_2$, respectively, and are subsequently summed.

Instead of using the distribution $P_1$ during the recordings, it is alternatively possible to use the distribution $P_2$ shown in Figure 2b. This distribution offers the advantage that it is disjunct, which means that each radiation source is used only once for the formation of coded images. The reconstruction of the object is again realized in four channels, because the distributions A and B can be composed from the four distributions $t_1$, $t_2$, $s_1$, $s_2$.

Moreover, on the basis of the distribution $P_1$ of Figure 2b, three recordings may also suffice if the complementary code pair has the property

$$g_1+g_2=h_1+h_2 \qquad (10)$$

which only means that the source positions of the two codes A and B correspond exactly.

It follows from (10) that:

$$h_2=g_1+g_2-h_1 \qquad (11)$$

so that

$$O_{h_2}=O_{g_1}+O_{g_2}-O_{h_1} \qquad (12)$$

Insertion of (12) in (9) produces

$$O''=(O_{g_1}-O_{g_2})\circledast g_1+(O_{g_2}-O_{g_1})\circledast g_2+ (2\,.\,O_{h_1}-O_{g_1}-O_{g_2})\circledast h_1+ (O_{g_1}+O_{g_2}-2.O_{h_1})\circledast h_2 \qquad (13)$$

so that only the three recordings $O_{g}$, $O_{g_2}$ and $O_{h_1}$ are required. Four channels are still required for the reconstruction.

The number of channels for the reconstruction can also be reduced to three, because it also follows from (10) that

$$g_2=h_1+h_2-g_1 \qquad (14)$$

and insertion of (14) in (13) results in

$$O''=(2\,.\,O_{g_1}-2\,.\,O_{g_2})\circledast g_1+(2\,.\,O_{h_1}-2\,.\,O_{g_1})\circledast h_1 +(2\,.\,O_{g_2}-2\,.\,O_{h_1})\circledast h_2 \qquad (15)$$

where only three recordings are made and only three channels are required for the reconstruction.

In addition to the described possibility, there are other possibilities of reducing the number of recordings and or channels for the reconstruction to three by way of (10). For example, the possibility can be chosen where in total the smallest number of sources is used for all exposures together. This is technically advantageous and it also means a lower radiation load for a patient to be irradiated.

The order of the complementary codes indicates how many X-ray tubes in a point source distribution are used for forming coded images of the object. The described method of coding and decoding is applicable to flat, two-dimensional objects. The layers of an arbitrary three-dimensional object can be decoded by changing the scale of the coded images as described in German Offenlegungsschrift 25.35.408.9 (=PHD 75—122c).

In order to find the complementary codes, the mathematical rules for forming the complementary codes are described hereinafter.

I. Properties of complementary codes

Some definitions

If A is a code, —A or the inverse code of A indicates the code were each +1 is changed into a —1 and *vice versa.* Two codes A and B are called disjunct if no position of a source of A corresponds to a position of a source of B. *The sum* of two disjunct codes A and B is denoted as A+B and is defined as the code produced by combination of A and B. Each source in A+B, therefore, was already a source in A or B where it had the same value +1 or —1. The sum of two codes A and —B is also indicated as the *difference* of A and B and is written as A—B.

Using these definitions, the following definitions can be demonstrated by simple algebra:

*Rule:* When A and B are complementary codes, the following codes are also complementary:

1) B and A.
2) A and —B.
3) A and any translation of B.
4) A and the rotation of B through 180°.
5) Rotations of A and B through the same angle.
6) Mirror-images of A and B with respect to the same axis.
7) Stretchings of A and B in the same direction and by the same factor (also <1 *i.e.* a compression).
8) Identical slanting of A and B.
9) A+B and A—B, if A and B are disjunct.

A large number of further rules can be deduced from these rules by the successive execution of some of said operations, utilizing the above rules 1 to 9.

II. Formation of complementary codes

Using a pair of complementary codes, new complementary codes having the same source numbers can be obtained by way of the rules 1 to 8. Using the rule 9, complementary code pairs having an increasingly larger number of sources can be step-wise obtained. The formation process is in general as follows:

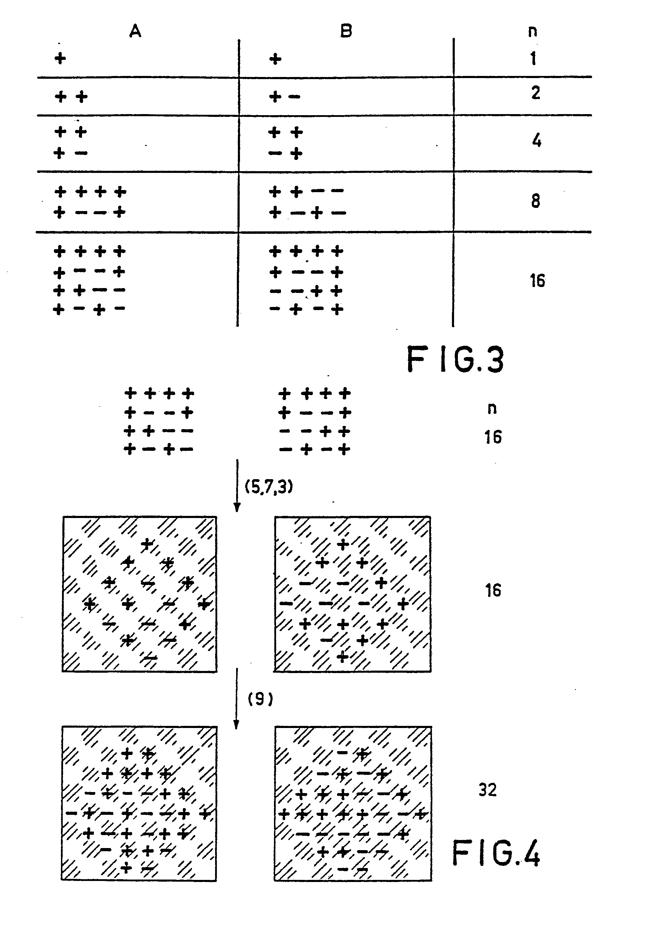

a) Take an already known pair of complementary codes (for example from the one-dimensional, see Figure 3).

b) Apply one of the rules 1 to 8 (also several times).
c) Apply rule 9.
d) Terminate the formation process with the newly obtained pair or continue at b).

An important combination is that of b) and c) which implies the *combination of* B with A and —B with A, consisting of rule 3 and 9, (generalization of the one-dimensional combination which converts a pair of complementary codes A, B, each of which comprises *n* sources, into a new pair of complementary codes, each of which comprises 2*n* sources).

A further possibility is the interleaving of A and B and of A and —B by way of the rule 3 (+ possibly twice the rule 7) and 9.

III. Concrete codes

Two-dimensional complementary codes can be obtained in accordance with the combination of rules (under II) as follows: Use is made of the codes A and B, each of which consists of only one source +1. First a horizontal combination of B with A and —B and A is performed, the codes thus produced being renamed A and B. Subsequently, a similar vertical combination is performed, followed by a horizontal combination etc. Thus, for each integer *n* a pair of complementary codes of the order $2^n$ is obtained and for each even *n* even source distributions which exactly form a square having the length and the width $2^{n/2}$. The first five pairs of complementary codes obtained according to this rule are shown in Figure 3.

IV. Invariability of complementary codes

The codes given in III and in Figure 3 represent only some examples for complementary pairs. Obviously, as is indicated sub II, complementary codes can also be derived from the rules given sub I in many other ways, notably by rotation, mirror-imaging stretching and slanting of the codes given in Figure 3.

The codes given therein, whose order is an odd power of 2 (2, 8, ...) have a rectangular shape instead of a square shape, the length of said rectangle being twice its width. However, using the general formation rule stated sub II, codes with a distribution in an approximately square area can also be indicated for these orders.

To this end, it is ensured by rotation through 45° (rule 5) and stretching by the factor 2 (rule 7) that two complementary codes occupy exactly the black fields of an (imaginary) checkerboard. Translation in the horizontal direction by one field to the left (rule 3) ensures that the first code occupies the black fields of the checkerboard and the second code occupies the white fields. Subsequently, the two codes are interleaved in accordance with rule 9, as described sub II.

When the codes used as a basis define a source distribution in a square area, such a distribution will also be defined by the codes produced by interleaving (in any case, the square is rotated through 45°). In figure 4 this principle for converting the described complementary code pair of the order 16 into a code pair of the order 32, which defines a source distribution in an almost square area, has been shown.

Using the same combination principle previously used for forming the complementary codes of the orders 1, 2, 4, 8, 16, new complementary code pairs can also be obtained, starting also with a different complementary pair of codes, and the positioning of the radiation sources for the image coding can be defined accordingly.

Hereinafter it is shown, by way of example, how from the pair of codes

+ + — and + . +

determined directly as a complementary pair and one of which has the order 3 and the other has the order 2, a series of complementary code pairs of the orders 5, 10, are built up. The combination is not always performed alternately vertically and horizontally, but such that always a source distribution in a square area is defined as well as possible.

The Figures 6*a*—*d* show radiation source distributions for codes of the order 3/2, 5, 10, 20, utilizing the combination rule 1) (Figure 6*b*), the rotation rule 4) (Figure 6*c*), and overlapping combinations (Figure 6*d*) being used. Figure 6*e* shows a regular matrix structure of equilateral triangles obtained by slanting through 30° to the left.

The arrangement shown in Figure 7 comprises four X-ray sources $Q_1$ to $Q_4$ which are arranged in one plane and which can be connected to a power supply + *via* diodes D and switches $S_1$ to $S_4$. When the switches $S_1$ to $S_4$ are operated, part of the sources $Q_1$ to $Q_4$ irradiate an object O, so that shadow images of the object O are formed on a positive film $F_1$ and a negative film $F_2$. For the sake of clarity, these shadow images are not shown. The two films $F_1$ and $F_2$ are arranged parallel to the plane in which the sources Q are arranged. The arrangement shown in Figure 7 corresponds to the radiation (point) source distribution shown in Figure 1. The radiation sources to be activated *via* the switches $S_1$ and $S_2$ form a code B. The radiation sources to be activated *via* the switches $S_3$ and $S_4$ form a code A. When the switch $S_1$ is closed for a brief period of time T, the object O is irradiated by the sources $Q_1$, $Q_2$ and $Q_4$. As described with reference to Figure 2, the coded images $O_{g_1}$ and $-O_{g_1}$ are recorded on the films $F_1$ and $F_2$. After replacement of the films $F_1$ and $F_2$, the switch $S_2$ is closed for the same period of time T, so that $O_{g_2}$ and $-O_{g_2}$ are recorded. Subsequently, the coded images $O_{h_1}$ and $-O_{h_1}$ and $O_{h_2}$ and $-O_{h_2}$, respectively, are recorded by the closing and opening of the switches $S_3$ and $S_4$ respectively.

The coded images $O_{g_1}$, $-O_{g_1}$, ... $O_{h_2}$ and $-O_{h_2}$, obtained by means of the arrangement described with reference to Figure 7, are decoded by means of an arrangement which will be described with reference to Figure 8. The arrangement shown comprises four optical channels, each of which is irradiated by one of four parallel beams of monochromatic light. The four light beams can be obtained, for example, by the splitting of a laser beam LA by means of semitransparent and fully reflective mirrors (see Figure 9). Each processing channel comprises a first lens system L1 for forming a wide light beam from the narrow laser beam in order to illuminate the coded images $O_{g_1}$ to $-O_{h_2}$. The light beams modulated by the coded images are projected on point holograms H, G *via* zoom lenses Z. Behind the point holograms $H_1$, $H_2$, $G_1$, $G_2$ there is arranged a projection screen S on which four light beams from the point holograms form a decoded image. The decoded image is an image of section of the object O (see Figure 7), said section being situated parallel to the plane in which the sources $Q_1$ to $Q_4$ are situated.

As will be clear from the formula (9), in the first channel the decoded (positive) image $O_{g_1}$ and the negative of the coded image $O_{g_2}$ are arranged. Using the point hologram $G_1$ of $G_1$, the sum of the two coded images $O_{g_1}$ and $-O_{g_2}$ is decoded. In the second channel there are situated the coded (positive) image $O_{g_2}$ and the negative $O_{g_1}$ which are decoded together with the point hologram $G_2$ of $g_2$. In the third channel there are situated the coded image $O_{h_1}$, the negative of the image $O_{h_2}$ and behind the zoom lens the point hologram $H_1$. Obviously, in the fourth channel there are situated the coded image $O_{h_2}$ and the negative of $O_{h_1}$ which are decoded *via* the point hologram $H_2$. The coded images $O_{g_1}$ to $O_{h_2}$ and the negatives thereof are pair-wise slid on a frame 10 which is arranged on a rigid support 1, which may be a slide frame holder. Similarly, there is provided a second frame 3 in which a (photo)sensitive film can be inserted for the recording of a decoded image. The lens system l1 and the point holograms $G_1$, $H_1$, $G_2$ and $H_2$ are each secured in a stationary frame 7 and 5, respectively, and require only one optical alignment operation, together with the zoom lenses Z which are mounted in a frame 9 which is displaceable parallel to the optical axis (direction indicated by arrows 11) of each channel for reasons yet to to be described. The arrangement briefly described above can be constructed by means of conventional optical means such as mirrors, lenses, lens holders, and other customarily used parts.

Figure 9 is a more detailed view of two optical channels of the arrangement of Figure 8. A laser LA is used to form a monochromatic light beam which is split into two parallel light beams by the mirrors $S_{T1}$ and $Sp_1$. Each beam in its turn is split into two further parallel beams, so that four parallel light beams are formed due to the use of the mirrors $Sp_2$ and $Sp_3$ and the semitransparent mirrors $S_{T2}$ and $S_{T3}$. The light beam is converted in each optical channel by a lens system $L_1$ into a wide beam of light which illuminates the entire coded image (for example, $O_{g_2}$ and $-O_{g_1}$). Instead of the large lens of the lens system $L_1$, use can alternatively be made of a frosted glass plate, the distance between the frosted glass plate and the coded images being as small as possible. The light transmitted by the coded image is converted, *via* a zoom lens system Z, into an intermediate image $A_1$, $A_2$, $A_3$, $A_4$ which is decoded *via* the imaging lens $L_2$ and the hologram $G_1$, $G_2$. The decoded image obtained *via* a channel is a sub-image which forms a complete (decoded) image S in conjunction with the three sub-images obtained *via* the further optical channels. Instead of using the imaging lens and the hologram (the point hologram or the multiplication hologram is the image point function of the group of sources with which the coded positive image arranged in the optical channel is formed), use can be made of a matrix of lenses, the position of the lenses in the matrix being determined by the image point function (the positions of the X-ray sources used for the recording).

It is to be noted that an arrangement for the decoding of coded images which comprises only two optical channels is already known from the German Offenlegungsschrift P 25.35.408.9.

Figure 10 shows a device for the coding of images and the decoding of coded images. The device comprises a holder 13 in which there are arranged a number of X-ray sources, an image intensifier 15, two decoding sections which are coupled to the exit window 21 *via* a fully reflective mirror 17 and a semitransparent mirror 19, each of said decoding sections comprising a lens matrix 23, 25, a diaphragm 27, 29, an image pick-up tube 31, 33, a differential amplifier 35, and an image display device 37 in the form of a television monitor.

The X-ray tubes arranged in the holder 13 are subdivided into three sub-groups, each of which can be connected to the power supply voltage + *via* an associated switch $S_5$, $S_6$, $S_7$. The sub-division into three subgroups is realized on the basis of the rule described with reference to Figure 2b ($P_1$). The switches $S_5$, $S_6$, $S_7$ are sequentially closed and opened, so that the coded images $O_{g_1}$, $O_{g_2}$ and $O_{h_1}$ of an object are projected onto the entrance screen 39 of the image intensifier 15 sequentially with the subgroups. The image received by the image intensifier is displayed on the entrance screen 21 introduced and intensified form. *Via* the semitransparent mirror 19, the coded image $O_{g_1}$, $O_{g_2}$ or $O_{h_1}$, is projected onto a rotating diaphragm 27. The diaphragm 27 leaves given parts of the lens matrix uncovered, as will be described hereinafter, so that the coded images $O_{g_1}$, $O_{g_2}$ and $O_{h_1}$ are decoded with the image point functions $g_1$, $h_2$, and $h_1$, respectively. The

decoded images are picked-up by the image pick-up tube 31.

The decoding of the images $O_{g_1}$, $O_{g_2}$ and $O_{h_1}$ with the correct image point function $g_1$, $h_2$ and $h_1$ is realized as follows. Each of the diaphragms 27 and 29 comprises three sectors. Each sector comprises slots 60 which enable the passage of the light to given lenses of the lens matrix in a given position of the diaphragm. The cooperation between the switches $S_5$, $S_6$, $S_7$, the diaphragms 27, 29 and the shape of the diaphragms will be described, by way of example, with reference to the source arrangement shown in Figure 7. As can be seen in formule (15), an image of a section of an object is obtained by a sum of three correlations. The formula 15 can be rewritten as:

$$O'' = 2 \cdot (O_{g_1} \circledast g_1 - O_{g_1} \circledast h_1) + 2 \cdot (O_{h_1} \circledast h_1 - O_{h_1} \circledast h_2) + 2 \cdot (O_{g_2} \circledast h_2 - O_{g_2} \circledast g_1) \quad (15')$$

When the object is exposed by means of the sources $Q_1$, $Q_2$ and $Q_4$ (subgroup with the image point function $g_1$) during a first exposure operation, the coded image $O_{g_1}$ (appearing on the exit window 21 of the image intensifier 15) is decoded with the function $g_1$ *via* the semitransparent mirror 19, the diaphragm 27 and the lens matrix 23. To this end, the diaphragm 27 comprises slits in a sector (see Figures 11*a* and *b*) which leave the location of the lenses in the matrix free during a third of the revolution of the diaphragm 27. *Via* the semitransparent mirror 19, the mirror 17, the diaphragm 29 and the lens matrix 25, the coded image $O_{g_1}$ is also decoded with the function $h_1$ (see Figures 11a and b). The decoded images are picked-up by means of the pick-up tubes 31 and 33 and are converted into an electric signal. The two electric signals thus obtained are applied to a differential amplifier 35, the output signal of which is applied to the monitor 37, so that the difference between the two signals:

$$2 \cdot (O_{g_1} \circledast g_1 - O_{g_1} \circledast h_1)$$

is displayed on the exit screen 41.

For a second exposure of the object, the sources $Q_1$, $Q_2$ and $Q_3$ are used. The coded image $O_{h_1}$ obtained *via* the image intensifier 15 is processed in the same way as the previously coded image $O_{g_1}$. The diaphragm 27 and 29, however, have been rotated one third of a revolution further, so that the coded image $O_{h_1}$ is decoded with the function $h_1$ *via* the diaphragm 27 and with the function $h_2$ *via* diaphragm 29. The decoded images are applied to the monitor *via* the pick-up tubes 31, 33 and the differential amplifier circuit 35 (the applied signal:

$$2 \cdot (O_{h_1} \circledast h_1 - O_{h_1} \circledast h_2).$$

For a third exposure of the object, only a single source $Q_4$ is used. Like the two previously coded images $O_{g_1}$ and $O_{h_1}$, the coded image $O_{g_2}$ is decoded *via* the lens matrices 23 and 25 and the diaphragms 27 and 29. The diaphragms are rotated a third of a revolution further again with respect with their position during the second exposure, so that now the coded image $O_{g_2}$ is decoded, *via* the diaphragm 27, with the function $g_1$ and the coded image $O_{g_2}$ is decoded with the function $h_2$ *via* the diaphragm 29. The decoded images are applied to the monitor 37 *via* the pick-up tubes and the differential amplifier circuit 35. The applied signal

$$2 \cdot (O_{g_2} \circledast g_1 - O_{g_2} \circledast h_2) (= 2 \cdot (O_{g_2} \circledast h_2 - O_{g_2} \circledast g_1)$$

has changed its sign, because the source $Q_4$ used is a "positive" source, whilst according to the calculation a "negative" source has to be used (which actually does not exist).

The third exposure is followed again by an exposure by means of the first group of X-ray sources $Q_1$, $Q_2$ and $Q_4$, for which the diaphragms 27 and 29 are rotated one third of a revolution further, *i.e.* back to their starting position. After the repeat of the first exposure, the second and the third irradiation are repeated etc.

If the image frequency (exposure frequency) is high enough, the successive images on the monitor screen will be observed as one image due to the inertia of the human eye. The use of an image screen with some after glow will also stimulate the "summing" of the successive images.

In order to synchronize the (continuous) rotation of the diaphragms with the exposure of the object and the reading of the pick-up tubes, near a diaphragm 29 there is arranged an optical detector 43 which per second supplies a number of pulses which are a measure for the (rotary) position of the diaphragms which are driven together (not shown). The pulse divider 45, being used as a counter, counts the number of pulses and supplies a pulse when a given position is reached. *Via* the control logic 49, whereto the pulse is applied, a switch $S_5$, $S_6$ or $S_7$ is briefly closed for a next exposure. The pulse is also applied to a deflection signal generator 47 in order to synchronize the reading of the two image pick-up tubes 31 and 33 and the display of the images read with the speed of revolution of the diaphragms.

Instead of using synchronously rotating diaphragms, use can also be made of stationary diaphragms, in which case electromechanical shutters are used for blocking the passage of light to a lens of the lens matrix or not.

The opening and closing of the shutters should then be synchronized, like the opening and closing of the switches $S_5$, $S_6$, $S_7$, for the successive exposures by means of the deflection signals (for example, for the monitor). *Via* a semitransparent mirror 51, the coded images successively formed on the exit screen 21 can be recorded by means of a film camera 53, a film image being made of each object image. To this end, the film camera 53 is coupled to the pulse divider 45 for the required synchronization pulse.

A film thus exposed can be introduced into the decoding array again (after development) by projecting the film against the rear of the mirror 51 as indicated by an arrow 55. As a result, a moving object can be studied, without it being necessary to expose this object each time to X-rays again.

Furthermore, between the mirrors 23 and 25 and the diaphragms 27 and 29 two coupled zoom lenses can be arranged (denoted by arrows 57 and 57') in order to enable reconstruction of more than one layer of the object as shown in Figure 9. For the sake of clarity, the zoom lenses are not shown in the Figure.

The diaphragms used in the device shown in Figure 10 are shown in the Figures 11*a* and *b*. Each diaphragm 27, 29 is formed by a round disc, for example, of aluminium which is divided into three sectors, in which some slits 60 which are shaped as an arc of a circle are provided. The slits 60 in each sector allow the passage of the light originating from the exit window of the image intensifier to some of the lenses in the matrix 23, 25 arranged behind the diaphragm (diagrammatically shown). Thus, during rotation of the diaphragms 27, 29, the coded image is decoded with a function which is determined by the sector of the disc which is situated in front of the lens matrix. As appears from the foregoing description, the diaphragm 27 should comprise three sectors which produce the functions $g_1$, $h_1$ and $g_1$ in conjunction with the lens matrix 23. The diaphragm 29 should comprise three sectors which produce the functions $h_1$, $h_2$ and $h_1$ in conjunction with the lens matrix 25. The edge of the disc is provided with slits 61 (only a few are shown) which produce pulses in conjunction with an optical sensor (Figure 10).

The method as claimed herein relates to a method of coding and decoding an image of an object. The result of the method, which is an image of a layer of an object, can be used in different ways. One way to use the image is in diagnosing a disease in a human or animal body. It is to be understood that the combination of the claimed method and the use of the result thereof for diagnosing a disease in a human or animal body is to be considered as a diagnostic method, which is excluded from patentability and not included within the scope of the present claims.

## Claims

1. A method of coding and decoding an image of an object by means of radiation which is emitted by point sources and which irradiates the object in different directions in order to form shadow images of the object which are superposed on a recording medium, the coded image thus formed being decoded by convolution of the superposed shadow images with a function which is determined by the point source distribution used for irradiating the object, characterized in that for the coding the point sources are arranged according to a regular matrix, at least three different groups of point sources being used to form a corresponding number of superposition images in succession, a combination of groups forming a first source distribution function and a further combination of groups forming a second source distribution function, the sum of the autocorrelation functions of said source distribution functions having the value zero except in one point (origin), and for the decoding the different superposition images, or combinations with the negatives thereof, are convoluted with image point functions of the groups of sources in order to obtain convoluted images which are summed in order to obtain a decoded image.

2. A method as claimed in Claim 1, characterized in that the radiation sources for forming coded images are used in four disjunct groups ($t_1$, $t_2$, $s_1$, $s_2$) which are successively activated, it being possible to compose the two distribution functions from said groups, a coded image formed by means of the first group of sources ($t_1$) being used as a positive in the first as well in the second distribution function, a coded image formed by means of the second group of sources ($t_2$) being used as a negative in the first as well as in the second distribution function, a coded image formed by means of the third group of sources ($s_1$) being used as a positive in the first distribution function and as a negative in the second distribution function, a coded image formed by means of the fourth group of sources ($s_2$ being used as a negative in the first distribution function and as a positive in the second distribution function, after which a sum of the positives of the coded images formed by means of the first and the third group of sources ($t_1$, $s_1$) and the negatives of the coded images formed by means of the second and the fourth group of sources ($t_2$, $s_2$) is convoluted with the image point function of the first group of sources $t_1$, thus producing a first sub-image, a sum of the negative and the positive of the images coded by means of the first and the second, respectively group of sources ($t_1$, $t_2$) being convoluted with the image point function of the second group of sources ($t_2$), thus forming a second sub-image, a sum of the positive and the negative of the images coded with the third and the fourth, respectively, group of sources ($s_1$, $s_2$) being convoluted with the image point function of the third group of sources ($s_1$), thus forming a third sub-image, a sum of the negative and the positive of the images coded with the third and the fourth, respectively group of sources ($s_1$, $s_2$) being con-

voluted with the image point function of the fourth group of sources ($s_2$), thus forming a fourth sub-image, the sum of the four sub-images forming a decoded image.

3. A method as claimed in Claim 1, characterized in that during the coding of the object with two complementary distribution functions of sources, each distribution function comprises all sources which are subdivided into different groups ($g_1$, $g_2$ and $h_1$, $h_2$) for each distribution function, the object being coded by irradiation by two groups ($g_1$, $g_2$) of a first distribution function and by only one group ($h_1$) of the second distribution function in order to obtain a positive as well as a negative of three images sequentially coded with the groups.

4. A method as claimed in Claim 3, characterized in that during the decoding

a. a first sum of a positive and a negative of the images coded by means of a first and the second group ($g_1$, $g_2$) respectively of sources associated with the first distribution function is convoluted with the image point function of the first group ($g_1$) of sources in order to obtain a first sub-image,
b. a negative of the first sum is convoluted with the image point function of the second group ($g_2$) of sources in order to obtain a second sub-image,
c. a second sum of two times a positive of the image coded by means of the group ($h_1$) of sources associated with the second distribution function and of the negatives of the images coded by means of the groups of the sources ($g_1$, $g_2$) associated with the first distribution function is convoluted with the image point function of the third group ($h_1$) in order to obtain a third sub-image,
d. a negative of the second sum is convoluted with an image point function which is determined by the difference between the image point function of all sources and the image point function of the third group of sources ($h_1$) in order to obtain a fourth sub-image, the sum of the four sub-images producing a decoded image.

5. A method as claimed in Claim 3, characterized in that during the decoding

a. the sum of a positive and a negative of the images coded by means of a first group ($g_1$) and a second group ($g_2$) of sources, respectively, which form the first distribution function is convoluted with the image point function of the first group ($g_1$) in order to obtain a first sub-image.
b. the sum of a positive and a negative of the images coded by means of the third group ($h_1$) and the first group ($g_1$), respectively, of sources being convoluted with the image point function of the third group in order to obtain a second sub-image,
c. the sum of a positive and a negative of the images coded by means of the second group ($g_2$) and the third group ($h_1$), respectively, of sources is convoluted with an image point function which is determined by the difference between the image point function of all sources and the image point function of the third group of sources ($h_1$) in order to obtain a third sub-image, the sum of the three sub-images forming a decoded image.

6. A method as claimed in Claim 3, characterized in that sequentially during the decoding

a. an image coded by means of a first group ($g_1$) of sources associated with the first distribution function is convoluted simultaneously and separately with the image point function of the first group ($g_1$) and with the image point function of a third group ($h_1$) of sources associated with the second distribution function, in order to obtain a first and a second, respectively, sub-image, said second sub-image being subtracted from the first sub-image in order to obtain a first difference image,
b. an image coded by means of the third group ($h_1$) of sources is simultaneously and separately convoluted with the image point functions of the third and the fourth, respectively group ($h_1$), ($h_2$) of sources associated with the second distribution function in order to obtain a third and a fourth sub-image, respectively, said fourth sub-image being subtracted from the third sub-image in order to obtain a second difference image,
c. an image coded by means of the second group ($g_2$) of sources of the first distribution function is simultaneously and separately convoluted with the image point functions of the first and the fourth group ($g_1$), ($h_2$) of sources, respectively, associated with the first and the second distribution function, respectively, in order to obtain a fifth and a sixth sub-image, said sixth sub-image being subtracted from the fifth sub-image in order to obtain a third difference image, after which the decoded image is obtained by the summing of the three difference images.

7. A device for coding and decoding an image of an object by means of penetrating radiation, said device comprising for the coding of an image of an object a two-dimensional point source distribution and a two-dimensional recording medium which are arranged in two parallel planes at a distance from each other in order to form superposition images of the object, said device comprising for the decoding of the superposition images means for the convoluting of the recorded superposition images which at least a part of an image point function which is defined by the point source distribution, and means for summing the convoluted superposition images, characterized in that the device for the coding comprises means for the sequential activation of the point sources in at least three and at the most four different groups in order to obtain at least three coded images, a first number of groups of point sources forming a first distribution function and a second number of groups forming a second distribution function, the sum of the autocorrelation of said distribution functions having the value zero with the exception of the value in one point (origin), the device for the decoding comprising at least three processing channels for convoluting, either sequentially or simultaneously, the coded images with corresponding image point functions, associated with the coded images, each of said functions being determined by different groups of point sources.

8. A device as claimed in Claim 7, characterized in that the means for the decoding of the coded images comprise at least three parallel operating processing channels, each channel comprising means for creating a two-dimensional, flat diffusely radiating light source, a holder which is arranged in front of the light source and which serves to accommodate at least one coded image, a lens and a holder for accommodating a point hologram of the point source distribution associated with the coded image, said means for the decoding furthermore comprising a projection screen on which a sub-image originating from each channel and generated *via* the point hologram is projected.

9. A device as claimed in Claim 6, characterized in that the means for the decoding of the coded images comprise at least three parallel operating processing channels, each channel comprising means for creating a two-dimensional flat diffuse radiation radiating light source, a holder which is arranged in front of the light source and which serves to accommodate at least one coded image and a holder for accommodating a matrix of lenses, the position of which in the matrix is determined by the positions of the point source distribution associated with the decoded image, said means for the decoding furthermore comprising a projection screen on which a sub-image originating from each channel and generated *via* the lens matrix is projected.

10. A device as claimed in Claim 7, characterized in that the means for the decoding comprise a two-dimensional radiation detector for forming, on the output thereof, a luminescent coded image, a semitransparent mirror and a fully reflective mirror which are coupled to the detector in order to obtain two identical, luminescent coded images, two assemblies of a matrix of lenses and a variable diaphragm for the separate optical convolution of the two luminescent images with the image point function associated with the images, two image pick-up devices, on a target of which the convoluted images are projected for the electronic scanning of these images, a differential stage which comprises two inputs whereto two outputs of the image pick-up devices are coupled in order to determine a difference between the two convoluted images, and a display/recording device for displaying the difference, the positions of the lenses in the matrix being determined by the positions of the point sources in the point source distribution, sequential activation of three groups of point sources being possible, for which each variable diaphragm is in three different conditions, each condition being determined by the activated group of point sources.

**Patentansprüche**

1. Verfahren zum Codieren und Decodieren eines Objektbildes mittels Strahlung, die von Punktquellen ausgestrahlt wird und die das Objekt zur Bildung von Schattenbildern des Objekts aus verschiedenen Richtungen durchstrahlt, welche Schattenbilder auf einem Aufzeichnungsträger überlagert werden, wobei das so geformte codierte Bild durch Faltung der überlagerten Schattenbilder mit einer Funktion decodiert wird, die durch die Punktquellenverteilung für die Bestrahlung des Objekts bestimmt wird, dadurch gekennzeichnet, dass für die Codierung die Punktquellen entsprechend einer regelmässigen Matrix angeordnet sind, wobei zumindest drei verschiedene Punktquellengruppen zur Bildung einer entsprechenden Anzahl aufeinanderfolgender Überlagerungsbilder verwendet werden, wobei eine Gruppenkombination eine erste Quellenverteilungsfunktion und eine weitere Gruppenkombination eine zweite Quellenverteilungsfunktion bilden, wobei die Summe der Autokorrelationsfunktionen der erwähnten Quellenverteilungsfunktionen ausser in einem Punkt (Ursprung) den Wert Null hat und dass für die Decodierung die verschiedenen Überlagerungsbilder oder Kombinationen mit ihren Negativen mit den Bildpunktfunktionen der Quellengruppen zum Erhalten gefalteter Bilder gefaltet werden.

2. Verfahren nach Anspruch 1, dadurch gekennzeichnet, dass die Strahlungsquellen zur Formung codierter Bilder in vier disjunkten Gruppen ($t_1$, $t_2$, $s_1$, $s_2$) verwendet werden, die nacheinander aktiviert werden, wobei es möglich ist, die zwei Verteilungsfunktionen aus diesen Gruppen zusammenzusetzten, wobei ein mit der ersten Quellengruppe ($t_1$) geformtes codiertes Bild sowohl in der ersten als auch in der zweiten Verteilungsfunktion als Positive benutzt wird, ein mit der zweiten Quellengruppe ($t_2$) geformtes codiertes Bild sowohl in der ersten als auch in der zweiten Vertielungsfunktion als Negativ benutzt wird, ein mit der dritten Quellengruppe ($s_1$) geformts codiertes Bild in der ersten Verteilungsfunktion als Positiv und in der zweiten Verteilungsfunktion als Negativ benutzt wird, ein mit der vierten Quellengruppe ($s_2$) geformtes codiertes Bild in der ersten Verteilungsfunktion als Negativ und in der zweiten Verteilungsfunktion als Positiv benutzt wird, wonach eine Summe der Positiven der mit den ersten und dritten Quellengruppen ($t_1$, $s_1$) geformten codierten Bilder und der Negativen der mit den zweiten und vierten Quellengruppen ($t_2$, $s_2$) geformten codierten Bilder mit der Bildpunktfunktion der ersten Quellengruppe $t_1$ gefaltet wird, wodurch ein erstes Unterbild erzeugt wird, wobei eine Summe des Negatives und des Positivs der mit der ersten bzw. zweiten Quellengruppe ($t_1$, $t_2$) codierten Bilder mit der Bildpunkt funktion der zweiten Quellengruppe ($t_2$) gefaltet wird und so ein zweites Unterbild formt, wobei eine Summe des Positivs und des Negativs der mit der dritten bzw. vierten Quellengruppe ($s_1$, $s_2$) codierten Bilder mit der Bildpunktfunktion der dritten Quellengruppe ($s_1$) gefaltet wird und so ein drittes Unterbild formt, wobei eine Summe des Negatives und des Positivs der mit der dritten bzw. vierten Quellengruppe ($s_1$, $s_2$) codierten Bilder mit der Bildpunktfunktion der vierten Quellengruppe ($s_2$) gefaltet wird und so ein viertes Unterbild formt, wobei die Summe der vier Unterbilder ein decodiertes Bild formt.

3. Verfahren nach Anspruch 1, dadurch gekennzeichnet, dass bei der Codierung des Objekts mit zwei komplementären Quellenverteilungsfunktionen jede Verteilungsfuntion alle Quellen umfasst, die in verschiedene Gruppen ($g_1$, $g_2$ und $h_1$, $h_2$) für jede Verteilungsfunktion unterteile sind, wobei das Objekt mittels Durchstrahlung durch zwei Gruppen ($g_1$, $g_2$) einer ersten Verteilungsfunktion und durch nur eine Gruppe ($h_1$) der zweiten Verteilungsfunktion zum Erhalten sowohl eines Positivs als auch eines Negativs von drei mit den Gruppen aufeinanderfolgend codierten Bildern codiert wird.

4. Verfahren nach Anspruch 3, dadurch gekennzeichnet, dass beim Decodieren

a. eine erste Summe eines Positivs und eines Negativs der mit der ersten bzw. zweiten Quellengruppe ($g_1$, $g_2$) codierten Bilder, welche Gruppen der ersten Verteilungsfunktion zugeodnet sind, mit der Bildpunktfunktion der ersten Quellengruppe ($g_1$) zum Erhalten eines ersten Unterbildes gefaltet wird,
b. ein Negativ der ersten Summe mit der Bildpunktfunktion der zweiten Quellengruppe ($g_2$) zum Erhalten eines zweiten Unterbildes gefaltet wird,
c. eine zweite Summe eines zweifachen Positivs des mit der der zweiten Verteilungsfunktion zugeordneten Quellengruppe ($h_1$) codierten Bildes und der Negative der mit den der ersten Verteilungsfunktion zugeordneten Quellengruppen ($g_1$, $g_2$) codierten Bilder mit der Bildpunktfunktion der dritten Gruppe ($h_1$) zum Erhalten eines dritten Unterbildes gefaltet wird,
d. ein Negativ der zweiten Summe mit einer Bildpunktfunktion gefaltet wird, die vom Unterschied zwischen der Bildpunktfunktion aller Quellen und der Bildpunktfunktion der dritten Quellengruppe ($h_1$) zum Erhalten eines vierten Unterbildes besitmmt wird, wobei die Summe der vier Unterbilder ein decodiertes Bild liefert.

5. Verfahren nach Anspruch 3, dadurch gekennzeichnet, dass beim Decodieren

a. die Summe eines Positivs und eines Negativs der mit einer ersten Quellengruppe ($g_1$) bzw. einer zweiten Quellengruppe ($g_2$), die die ersten Verteilungsfunktion bilden, codierten Bilder mit der Bildpunktfunktion der ersten Gruppe ($g_1$) zum Erhalten eines ersten Unterbildes gefaltet wird,
b. die Summe eines Positivs und eines Negativs der mit der dritten Quellengruppe ($h_1$) bzw. der ersten Quellengruppe ($g_1$) codierten Bilder mit der Bildpunktfunktion der dritten Gruppe zum Erhalten eines zweiten Unterbildes gefaltet wird,
c. die Summe eines Positivs und eines Negatives der mit der zweiten Quellengruppe ($g_2$) bzw. der dritten Quellengruppe ($h_1$) codierten Bilder mit einer Bildpunkfunktion, die vom Unterschied zwischen der Bildpunktfunktion aller Quellen und der Bildpunktfunktion der dritten Quellengruppe ($h_1$) bestimmt wird, zum Erhalten eines dritten Unterbildes gefaltet wird, wobei die Summe der drei Unterbilder ein decodiertes Bild formt.

6. Verfahren nach Anspruch 3, dadurch gekennzeichnet, dass aufeinanderfolgend bei der Decodierung

a. ein mit einer ersten Quellengruppe ($g_1$) codiertes Bild, welche Gruppe der ersten Vertielungsfunktion zugeordnet ist, gleichzeitig und getrennt mit der Bildpunktfunktion der ersten Gruppe ($g_1$) und mit der Bildpunktfunktion einer dritten Quellengruppe ($h_1$), die der zweiten Verteilungsfunktion zugeordnet ist, zum Erhalten eines ersten bzw. eines zweiten Unterbildes gefaltet wird, wobei das zweite Unterbild vom ersten Unterbild zum Erhalten eines ersten Differenzbildes subtrahiert wird,
b. ein mit der dritten Quellengruppe ($h_1$) codiertes Bild gleichzeitig und getrennt mit den Bildpunktfunktionen der dritten bzw. der vierten Quellengruppe ($h_1$ bzw. $h_2$) die der zweiten Verteilungsfunktion zugeordnet sind, zum Erhalten eines dritten bzw. eines vierten Unterbildes gefaltet wird, wobei das vierte Unterbild vom dritten Unterbild zum Erhalten eines zweiten Differenzbildes subtrahiert wird,
c. ein mit der zweiten Quellengruppe ($g_2$) der ersten Verteilungsfunktion codiertes Bild gleichzeitig und getrennt mit den Bildpunktfunktionen der ersten bzw. der vierte Quellengruppe ($g_1$ bzw. $h_2$), die der ersten bzw. der zweiten Verteilungsfunktion zugeordnet sind, zum Erhalten eines fünften und eines sechsten Unterbildes gefaltet wird, wobei das sechste Unterbild vom fünften Unterbild zum Erhalten eines dritten Differenzbildes subtrahiert wird, wonach das decodierte Bild durch die Summierung der drei Differenzbilder erhalten wird.

7. Gerät zum Codieren und Decodieren eines Objektbildes mittels durchdringender Strahlung, das zum Codieren eines Objektbildes eine zweidimensionale Punktquellenverteilung und einen zweidimensionalen Aufzeichnungsträger enthält, die in zwei parallelel Ebenen im Abstand voneinander zum Formen von Überlagerungsbildern des Objekts angeordnet sind, welches Gerät für die Decodierung der Überlagerungsbilder Faltungsmittel für die aufgezeichneten Überlagerungsbilder mit zumindest einem Teil einer Bildpunktfunktion, die von der Punktquellenverteilung bestimmt ist, und Summierungsmittel für die gefalteten Überlagerungsbilder enthält, dadurch gekennzeichnet, dass das Gerät zum Codieren Mittel für die aufeinanderfolgende Aktivierung der Punktquellen in zumindest drei und höchstens vier verschiedenen Gruppen enthält, um zumindest drei codierte Bilder zu erhalten, wobei eine erste Punktquellengruppe eine erste Verteilungsfunktion und eine zweiten Gruppe eine zweite Verteilungsfunktion bilder, wobei die Summe der Autokorrelation der Verteilungsfunktionen mit Ausnahme des Werts in einem Punkt (Ursprung) den Wert Null haben, wobei das Gerät für die Decodierung Bearbeitungsmittel zum aufeinanderfolgenden oder zum gleichzeitigen Falten der codierten Bilder mit entsprechenden Bildpunktfunktionen enthält, die den codierten Bildern zugeordnet sind, wobei eine jede der Funktionen durch verschiedene Punktquellengruppen bestimmt werden.

8. Gerät nach Anspruch 7, dadurch gekennzeichnet, dass die Mittel zum Decodieren der codierten Bilder zumindest drei parallel arbeitende Bearbeitungskanäle enthalten, wobei jeder Kanal Mittel zum Schaffen einer zweidimensionalen, flachen, diffus strahlenden Lichtquelle, eine vor der Lichtquelle angeordnete Halterung zum Aufnehmen zumindest eines codierten Bildes, eine Linse und eine Halterung zum Aufnehmen eines dem codierten Bildes zugeordneten Punkthologramms der Punktquellenverteilung enthält, wobei die Mittel für die Decodierung weiter einen Projektionsschirm enhalten, auf dem ein aus jedem Kanal herrührendes und über Punkthologramm erzeugtes Unterbild projiziet wird.

9. Gerät nach Anspruch 6, dadurch gekennzeichnet, dass die Mittel zum Decodieren der codierten Bilder zumindest drei parallel arbeitende Bearbeitungskanäle enthalten, wobei jeder Kanal Mittel zum Schaffen einer zweidimensionalen, flachen, diffuse Strahlung aussendenden Lichtquelle, eine vor der Lichtquelle angeordnete Halterung zum Aufnehmen zumindest eines codierten Bildes und eine Halterung zum Aufnehmen einer Linsenmatrix enthält, wobei die Positionen der Linsen in der Matrix durch die Positionen der dem decodierten Bild zugeordneten Punktquellenverteilung bestimmt werden, wobei die Mittel zum Decodieren weiter einen Porjektionsschirm enthalten, auf dem ein aus jedem Kanal herrührendes und über die Linsematrix erzeugtes Unterbild projiziert wird.

10. Gerät nach Anspruch 7, dadurch gekennzeichnet, dass die Mittel zum Decodieren einen zweidimensionalen Detektor zur Formung eines leuchtenden codierten Bildes an seinem Ausgang, einen halbdurchlässigen Spiegel und einen ganz reflektierenden Spiegel, die zum Erhalten zweier gleicher, leuchtender codierter Bilder mit dem Detektor verbunden sind, zwei Einrichtungen einer Linsenmatrix und einer änderbaren Blende für die getrennte optische Faltung der zwei leuchtenden Bilder mit der den Bildern zugeordneten Bildpunktfunktion, zwei Bildaufnahmegeräte, bei denen auf einem Target die gefalteten Bilder für die elektronische Abtastung dieser Bilder projiziert werden, eine Differentialstufe mit zwei Eingängen, an die zwei Ausgänge der Bildaufnahmegeräte zum Bestimmen einer Differenz zwischen den zwei gefalteten Bildern angeschlossen sind, und ein Aufnahme/Wiedergabe-Gerät für die Wiedergabe der Differenz enthalten, wobei die Positionen der Linsen in der Matrix durch die Positionen der Punktquellen in der Punktquellenverteilung bestimmt werden, wobei aufeinanderfolgende Aktivierung von drei Punktquellengruppen möglich ist, für die jede änderbare Blende drei verschiedene Zustände hat, wobei jeder Zustand durch die aktivierte Punktquellengruppe bestimmt wird.

**Revendications**

1. Procédé pour le codage et le décodage d'images d'un objet au moyen d'une radiation émise par des sources ponctuelles et pénétrant dans l'objet suivant des directions différentes avec laquelle des images d'ombre de l'objet sont enregistrées en superposition sur un milieu d'enregistrement, alors que par convolution de ces images d'ombre superposées avec une fonction que est déterminée par une répartition de sources ponctuelles utilisée lors de l'irradiation de l'objet, lesdites images superposées sont décodées, caractérisé en ce que les sources ponctuelles sont placées sur une grille régulière alors qu'avec au moins trois groupes différents de sources ponctuelles il est formé un nombre d'images de superposition qui correspond au nombre de groupes, une combinaison de groupes formant une première fonction de répartition alors qu'une autre combinaison de groupes forme une deuxième fonction de répartition, et que la somme des fonctions d'autocorrélation desdites fonctions de répartition a la valeur zéro exception faite de la valeur dans un seul point (l'origine, après quoi les différentes images de superposition ou des combinaisons avec les négatifs de ces images sont soumises à convolution avec des fonctions de points d'image des groupes de sources pour l'obtention d'images convoluées, celles-ci étant additionnées pour l'obtention d'une image décodée.

2. Procédé selon la revendication 1, caractérisé en ce que les sources de rayonnement pour former des images codées sont subdivisées en quatre groupes disjoints ($t_1$, $t_2$, $s_1$, $s_2$) qui sont utilisés consécutivement et à partir desquels il est possible de composer deux fonctions de répartition, alors qu'une image codés formée au moyen du premier groupe ($t_1$) de sources est utilisée comme positif aussi bien dans la première fonction de répartition que dans la deuxième fonction de répartition, qu'une image codée formée au moyen du deuxième groupe ($t_2$) de sources est utilisée comme négatif aussi bien dans la première fonction de répartition que dans la deuxième fonction de répartition, qu'une image codée formée au moyen de troisième groupe ($s_1$) de sources est utilisée comme positif dans la première fonction de répartition et comme négatif dans la deuxième fonction de répartition et qu'enfin une image codée formée au moyen du quatrième groupe ($s_2$) de sources est utilisée comme négatif dans la première fonction de répartition et comme positif dans la deuxième fonction de répartition, après quoi une somme des positifs des images codées formées au moyen des premier et troisième groupes de sources ($t_1$, $s_1$) et des négatifs des images codées formées au moyen des deuxième et quatrième groupes de sources ($t_2$, $s_2$) est convoluée avec la fonction de points d'image du premier groupe de sources ($t_1$) et fournit aussi une première image partielle, une somme du négatif et du positif des images codées formées au moyen des premier et deuxième groupes de sources ($t_1$, $t_2$) est convoluée avec la fonction de points d'image du deuxième groupe de sources ($t_2$) et fournit ainsi une deuxième image partielle, une somme du positif et du négatif des images codées formées au moyen des troisième et quatrième groupes de sources ($s_1$, $s_2$) est convoluée avec la fonction de points d'image du troisième groupe de sources ($s_1$) et fournit ainsi une troisième image partielle, une somme du négatif et du positif des images codées formées au moyen des troisième et quatrième groupes de sources ($s_1$, $s_2$) est convoluée avc la fonction de points d'image du quatrième groupe de sources ($s_2$) et fournit ainsi une quatrième image partielle, alors qu'enfin la somme desdites quatre images partielles fournit une image décodée.

3. Procédé selon la revendication 1, caractérisé en ce que lors du codage de l'objet avec deux fonctions de répartition complémentaires de sources, chaque fonction de répartition comprend toutes les sources qui pour chaque fonction de répartition sont subdivisées en deux groupes différents ($g_1$, $g_2$ et $h_1$, $h_2$), alors que pour le codage de l'objet ce dernier est irradié par deux groupes ($g_1$, $g_2$) d'une première fonction de répartition et par seulement un groupe ($h_1$) de la deuxième fonction de répartition pour obtenir aussi bien un positif qu'un négatif de trois images codées séquentiellement avec les groupes.

4. Procédé selon la revendication 3, caractérisé en ce que lors du décodages

*a* une première somme d'un positif et d'un négatif des images codées au moyen des premier et deuxième groupes de sources ($g_1$, $g_2$) appartenant à la première fonction de répartition est convoluée avec la fonction de points d'image du premier groupe de source ($g_1$) pour l'obtention d'une première image partielle,

*b* un négatif de la première somme est convolué avec la fonction de points d'image du deuxième groupe de sources ($g_2$) pour l'obtention d'une deuxième image partielle,

*c* une deuxième somme de deux fois un positif de l'image codée au moyen du groupe de sources ($h_1$) appartenant à la deuxième fonction de répartition et des négatifs des images codées au moyen des groupes de sources ($g_1$, $g_2$) appartenant à la première fonction de répartition est convoluée avec la fonction de points d'image du troisième groupe de sources ($h_1$) pour l'obtention d'une troisième image partielle, et enfin

*d* un négatif de la deuxième somme est convolué avec une fonction de points d'image qui est défini par la différence entre la fonction de points d'image de toutes les sources de rayonnement et la fonction de points d'image du troisième groupe de sources ($h_1$) pour l'obtention d'une quatrième image partielle, et qu'enfin la somme desdites quatre images partielles fournit une image décodée.

5. Procédé selon la revendication 3, caractérisé en ce que lors du décodage

*a* la somme d'un positif et d'un négatif des images codées au moyen d'un premier groupe de sources ($g_1$) et d'un deuxième groupe de sources ($g_2$) qui forment la première fonction de répartition est convoluée avec la fonction dé points d'image du premier groupe ($g_1$) pour l'obtention d'une première image partielle,

*b* la somme d'une positif et d'un négatif des images codées au moyen d'un troiséme groupe de source ($h_1$) et du premier groupe de sources ($g_1$) est convoluée avec la fonction de points d'image du troisième groupe de sources ($h_1$) pour l'obtention d'une deuxième image partielle,

*c* la somme d'un positif et d'un négatif des images codées formées au moyen du deuxième groupe de sources ($g_2$) et du troisième groupe de sources ($h_1$) est convoluée avec une fonction de points d'image qui est définie par la différence entre la fonction de points d'image de toutes les sources de rayonnement et la fonction de points d'image du troisième groupe de sources ($h_1$) pour l'obtention d'une troisième image partielle, alors qu'enfin la somme desdites trois images partielles fournit une image décodée.

6. Procédé selon la revendication 3, caractérisé en ce que de façon séquentielle lors du décodage

*a* une image codée au moyen d'un premier groupe de sources ($g_1$) appartenant à la première fonction de répartition est convolué simultanément et séparément avec la fonction de points d'image du premier groupe de sources ($g_1$) et avec la fonction de points d'image d'un troisième groupe de sources ($h_1$) appartenant à la deuxième fonction de répartition pour l'obtention de première et deuxième images partielles, le deuxième image partielle étant soustraite de première image partielle pour obtenir une première image de différence,

*b* une image codée au moyen du troisième groupe de sources ($h_1$) est convoluée simultanément et séparément avec les fonctions de points d'image du troisième et quatrième groupes de sources ($h_1$), ($h_2$) appartenent à la deuxième fonction de répartition pour l'obtention de troisième et quatrième images partielles, la quatrième image partielle étant soustraite de la troisième image partielle pour obtenir une deuxième image de différence,

*c* une image codée au moyen du deuxième groupe de sources ($h_2$) appartenant à la première fonction de répartition est convoluée simultanément et séparément avec les fonctions de points d'image des premier et quatrième groupes de sources ($g_1$), ($h_2$) appartenant respectivement au première et deuxième fonctions de répartition pour l'obtention de cinquième et sixième image partielles, la sixième image partielle étant soustraite de la cinquième image partielle pour obtenir une troisième image de différence, alors qu'enfin la sommation des trois image de différence fournit une image décodée.

7. Dispositif pour le codage et de décodage d'images d'un objet au moyen d'une radiation pénetrante, ce dispositif comportant pour le codage d'une image de l'objet une répartition bidimensionnelle de sources ponctuelles et un milieu bidimensionnel d'enregistrement qui sont placés à une certaine distance l'un de l'autre pour la formation d'image de superposition de l'objet, alors que pour le décodage des images de superposition de l'objet, ledit dispositif comporte des moyens pour la convolution des images de superposition enregistrée avec au moins une partie d'une fonction de points d'image définie par la répartition de sources ponctuelles, ainsi que des moyen pour la sommation des images de superposition soumises à convolution, caractérisé en ce que pour réaliser le codage, ce dispositif comporte des moyens pour activer de façon séquentielle les sources ponctuelles en au moins trois et au maximum quatre groupe différents pour l'obtention d'au moins trois images codées, alors qu'un premier nombre de groupes de sources ponctuelles forme une première fonction de répartition et qu'un deuxième nombre de groupes de sources ponctuelles forme une deuxième fonction de répartition, la somme des autocorrélations de ces fonctions ayant la valeur zéro exception faite de la valeur dans un point (l'origine), tandis que pour réaliser le décodage ledit dispositif comporte des moyens pour effectuer soit séquentiellement soit simultanément la convolution des images codées avec des fonctions de points d'image appartenant aux images codées, alors que chaque fonction de points d'image est définie par un différent groupe de sources ponctuelles.

8. Dispositif selon la revendication 7, caractérisé en ce que les moyen pour le décodage des images codées comportent au moins trois canaux de traitement que fonctionnent en parallèle et dont chacun est muni de moyens pour l'engendrement d'une source plane bidimensionelle de lumière diffuse, un support que est placé devant la source de lumière et sert à recevoir au moins une image codée, une lentille ainsi qu'un support pour recevoir un hologramme de points de la répartition de sources ponctuelles apparenant à l'image codée, alors que lesdits moyens de décodage comportent encore un écran de projection sur lequel est projetée une image partielle qui, par l'intermédiaire de l'hologramme de points, provient de chaque canal de traitement.

9. Dispositif selon la revendication 6, caractérisé en ce que les moyens pour le décodage des images codées comportent au moins trois canaux de traitement qui fonctionnent en parallèle et dont chacun est muni de moyens pour l'engendrement d'une source plane bidimensionnele de lumière diffuse, un support que est placé devant la source de lumière et sert à recevoir au moins une image codée, ainsi qu'un support pour recevoir un ensemble matriciel de lentilles, la position de ces lentilles dans leur ensemble matriciel étant définie par les positions de la répartition de sources ponctuelles appartenant à l'image codée, alors que lesdits moyens de décodage comportent encore un écran de projection sur lequel est projétée une image partielle qui, par l'intermédiaire de l'ensemble matriciel de lentilles, provient de chaque canal de traitement.

10. Dispositif selon la revendication 7, caractérisé en ce que les moyens pour le décodage comportent un détecteur de rayonnement bidimensionnel pour former sur la sortie de ce détecteur une image codée lumineuse, un miroir semiperméable et un miroir à réflexion totale qui sont couplés audit détecteur pour l'obtention de deux images codées lumineuses identiques, deux ensembles matriciels de lentilles combiné avec deux diaphragmes variables pour convoluer optiquement et séparément les deux images lumineuses avec la fonction de points d'image appartenent à ces images, deux dispositifs d'enregistrement d'image avec une cible sur laquelle a lieu la projection des images convoluées pour la lecture électronique de ces images, un étage de différence avec deux entrées auxquelles sont couplées deux sorties desdits dispositifs d'enregistrement d'image pour determiner ainsi une différence entre les deux imges convoluées, ainsi qu'un dispositif de reproduction/enregistrement pour reproduire ladite différence, la position des lentilles dans les ensemble matriciels de lentilles étant définie par les positions des sources ponctuelles dans la répartition de sources ponctuelles alors que de façon séquentielle il est possible d'activer trois groupes de sources ponctuelles, tandis que chaque diaphragme variable peut se trouver dans trois situations différentes dont chacune est définie par le groupe de sources ponctuelles en action.

| + | + |
|---|---|
| + | − |

A

| + | + |
|---|---|
| − | + |

B

FIG.1a

| -1 | 0 | 1 |
|---|---|---|
| 0 | 4 | 0 |
| 1 | 0 | -1 |

$A \circledast A$

| 1 | 0 | -1 |
|---|---|---|
| 0 | 4 | 0 |
| -1 | 0 | 1 |

$B \circledast B$

| 0 | 0 | 0 |
|---|---|---|
| 0 | 8 | 0 |
| 0 | 0 | 0 |

$0'' = 0 \circledast (A \circledast A + B \circledast B)$

FIG.1b

| + | + | + | + |
|---|---|---|---|
| + | − | − | + |
| + | + | − | − |
| + | − | + | − |

A

| + | + | + | + |
|---|---|---|---|
| + | − | − | + |
| − | − | + | + |
| − | + | − | + |

B

FIG.2a

$g_1$

| + | + | + | + |
|---|---|---|---|
| + | · | · | + |
| + | + | · | · |
| + | · | + | · |

$h_1$

| + | + | + | + |
|---|---|---|---|
| + | · | · | + |
| · | · | + | + |
| · | + | · | + |

$t_1$

| + | + | + | + |
|---|---|---|---|
| + | · | · | + |
| · | · | · | · |
| · | · | · | · |

$s_1$

| · | · | · | · |
|---|---|---|---|
| · | · | · | · |
| + | + | · | · |
| + | · | + | · |

$g_2$

| · | · | · | · |
|---|---|---|---|
| · | − | − | · |
| · | · | − | − |
| · | − | · | − |

$h_2$

| · | · | · | · |
|---|---|---|---|
| · | − | − | · |
| − | − | · | · |
| − | · | − | · |

$t_2$

| · | · | · | · |
|---|---|---|---|
| · | − | − | · |
| · | · | · | · |
| · | · | · | · |

$s_2$

| · | · | · | · |
|---|---|---|---|
| · | · | · | · |
| · | · | − | − |
| · | − | · | − |

P1

$g_1 + g_2 = A$ $h_1 + h_2 = B$

P 2

$t_1 + t_2 + s_1 + s_2 = A$

$t_1 + t_2 - (s_1 + s_2) = B$

FIG.2b

| A | B | n |
|---|---|---|
| + | + | 1 |
| + + | + − | 2 |
| + +<br>+ − | + +<br>− + | 4 |
| + + + +<br>+ − − + | + + − −<br>+ − + − | 8 |
| + + + +<br>+ − − +<br>+ + − −<br>+ − + − | + + + +<br>+ − − +<br>− − + +<br>− + − + | 16 |

FIG.3


n
16
(5,7,3)
16
(9)
32


FIG.4

| A | B | n |
|---|---|---|
| + + − | + · + | 3/2 |
| + + −<br>+ · + | + + −<br>− · − | 5 |
| + + −<br>+ · +<br>+ + −<br>− · − | + + −<br>+ · +<br>− − +<br>+ · + | 10 |

FIG.5

A B n

+ + − + · + 3/2

a 5

b 5

c 10

d 20

e

FIG.6

Q1
Q2
Q4
Q3
D
D
S1
B
S2
S3
A
S4
O
F1
F2
P3
P4
P2
P1


FIG.7

Sp3
A4
Sp2
Og2
G1
L1
Og1
Z
A2
L2
Sp1
ST1
Z
A3
L2
S
LA
ST2
ST3
L1
Og2
Og1
A1
G2


FIG.9

S
3
H1
H2
G1
G2
Z
Z
10
5
9
11
Oh1-Oh2
Oh2-Oh1
Og1-Og2
Og1
Og2-Og1
1
L1
L1
L1
7

FIG. 8

13
+
S5
S6
S7
49
O
39
15
21
53
51
55
19
17
47
45
57
43
27
57
29
23
25
31
33
41
35
37

FIG. 10

60
h1
60
27
g1
23
60
g1

FIG.11a

h2
60
61
29
h1
25
60
h1

FIG.11b